# EUROPEAN PATENT SPECIFICATION

(11) **EP 3 911 732 B1**
(45) Date of publication and mention of the grant of the patent: **03.04.2024**
(21) Application number: 20701389.7
(22) Date of filing: 13.01.2020
(51) Int. Cl.: C12N 5/07, C12N 15/63

(54) **ISOLATION AND CULTIVATION OF MUSCLE AND FAT CELLS FROM CRUSTACEANS**
ISOLIERUNG UND KULTIVIERUNG VON MUSKEL- UND FETTZELLEN AUS KRUSTENTIEREN
ISOLEMENT ET CULTURE DE CELLULES MUSCULAIRES ET ADIPEUSES DE CRUSTACÉS

(30) Priority: 15.01.2019 SG 10201900357Q; 03.06.2019 US 201962856479 P
(43) Date of publication of application: 24.11.2021
(73) Proprietor: Shiok Meats Pte. Ltd., Singapore 619860 (SG)
(72) Inventor: SRIRAM, Sandhya, Singapore 619860 (SG); LING, Ka Yi, Singapore 619860 (SG)
(74) Representative: Elsy, David
(86) International application number: PCT/SG2020/050016
(87) International publication number: WO 2020/149791

(56) References cited:
- JIE MA ET AL: "Penaeid shrimp cell culture and its applications", REVIEWS IN AQUACULTURE, vol. 9, no. 1, 26 August 2015 (2015-08-26) , pages 88-98, XP055683626, ISSN: 1753-5123, DOI: 10.1111/raq.12106
- Huarong Guo: "In-vitro Cell Culture of Aquatic Invetebrates - Penaeus Shrimps", Journal of Cell and Developmental Biology 1(3), 22 September 2017 (2017-09-22), XP055683741, Retrieved from the Internet: URL:https://www.imedpub.com/articles/invit ro-cell-culture-of-aquatic-invetebrates--p enaeus-shrimps.pdf [retrieved on 2020-04-07]
- PUTHUMANA JAYESH ET AL: "Attempts on producing lymphoid cell line fromPenaeus monodonby induction with SV40-T and 12S EIA oncogenes", FISH AND SHELLFISH IMMUNOLOGY, ACADEMIC PRESS, LONDON, GB, vol. 47, no. 2, 14 August 2015 (2015-08-14), pages 655-663, XP029326609, ISSN: 1050-4648, DOI: 10.1016/J.FSI.2015.08.010
- THANSA KWANTA ET AL: "Optimisation of electroporation and lipofection protocols to derive the black tiger shrimp cell line (Penaeus monodon)", FISH AND SHELLFISH IMMUNOLOGY, ACADEMIC PRESS, LONDON, GB, vol. 81, 17 July 2018 (2018-07-17), pages 204-213, XP085444511, ISSN: 1050-4648, DOI: 10.1016/J.FSI.2018.07.030
- JAYESH P ET AL: "Impaired telomerase activity hinders proliferation and in vitro transformation ofPenaeus monodonlymphoid cells", CYTOTECHNOLOGY, KLUWER ACADEMIC PUBLISHERS, DORDRECHT, NL, vol. 68, no. 4, 18 June 2015 (2015-06-18), pages 1301-1314, XP036014217, ISSN: 0920-9069, DOI: 10.1007/S10616-015-9890-9 [retrieved on 2015-06-18]
- P. JAYESH ET AL: "Multifactorial interaction of growth factors on Penaeus monodon lymphoid cells and the impact of IGFs in DNA synthesis and metabolic activity in vitro", CYTOTECHNOLOGY., vol. 67, no. 3, 25 February 2014 (2014-02-25), pages 559-571, XP055683750, NL ISSN: 0920-9069, DOI: 10.1007/s10616-014-9697-0
- Natalie Rubio ET AL: "Cell-Based Fish: A Novel Approach to Seafood Production and an Opportunity for Cellular Agriculture", , 12 November 2018 (2018-11-12), XP055683753, DOI: 10.20944/preprints201811.0326.v1 Retrieved from the Internet: URL:https://www.preprints.org/manuscript/2 01811.0326/v1
- Kazutoshi Takahashi ET AL: "Induction of Pluripotent Stem Cells from Adult Human Fibroblasts by Defined Factors", Cell, vol. 131, no. 5, 1 November 2007 (2007-11-01), pages 861-872, XP055547222, Amsterdam NL ISSN: 0092-8674, DOI: 10.1016/j.cell.2007.11.019
- Slamecka Jaroslav ET AL: "Non-integrating episomal plasmid-based reprogramming of human amniotic fluid stem cells into induced pluripotent stem cells in chemically defined conditions", CELL CYCLE, vol. 15, no. 2, 17 January 2016 (2016-01-17), pages 234-249, XP055939692, US ISSN: 1538-4101, DOI: 10.1080/15384101.2015.1121332
- Stepanyan R.: "Primary Culture of Lobster (Homarus americanus) Olfactory Sensory Neurons", Chemical Senses, vol. 29, no. 3, 1 March 2004 (2004-03-01), pages 179-187, XP055939695, DOI: 10.1093/chemse/bjh023
- BUNNELL B A ET AL: "Adipose-derived stem cells: Isolation, expansion and differentiation", METHODS, ACADEMIC PRESS, NL, vol. 45, no. 2, 1 June 2008 (2008-06-01), pages 115-120, XP022796172, ISSN: 1046-2023, DOI: 10.1016/J.YMETH.2008.03.006 [retrieved on 2008-05-29]
- Hedrick R. P. ET AL: "Adenovirus-Like Particles Associated with a Disease of Cultured White Sturgeon, Acipenser transmontanus", CANADIAN JOURNAL OF FISHERIES AND AQUATIC SCIENCES, vol. 42, no. 7, 1 July 1985 (1985-07-01), pages 1321-1325, XP055939700, CA ISSN: 0706-652X, DOI: 10.1139/f85-165

## Description

### FIELD OF THE INVENTION

The present disclosure is directed to the formation of renewable crustacean stem cell lines comprising muscle stem cells and/or fat stem cell lines from shrimp, prawn, crab, crayfish, and/or lobster species. A method for producing a shrimp or prawn renewable muscle stem cell line is also provided.

### BACKGROUND OF THE INVENTION

The world population continues to increase while the amount of arable agricultural land decreases due to urban sprawl and changing weather conditions. This has increased commercial fishing and ocean "farming," putting stress on the world's seas and oceans. As a result, overfishing of particular popular species such as shrimp, prawn, crab, and lobster has significantly decreased those wild populations.

In an attempt to ease the strain that animal meat production and wild fishing have on the environment, techniques are being developed for tissue engineered meats and fish. This type of cellular agriculture permits the production of food without the need to grow and sacrifice animals. Most of the research in cellular agriculture focuses on animals that have a wealth of information available from studies of embryonic muscle development, muscle stem cell, muscle repair, and muscle regeneration - in particular beef, pork, and chicken. Many challenges exist, however, in producing a meat or fish product that is a "like-for-like" replica of muscle extracted from a living animal.

However, for seafood species, little research has been done, compounding the obstacles which must be overcome for successful cellular agricultural work (Rubio et al., (2019) "Cell-Based Fish: A Novel Approach to Seafood Production and an Opportunity for Cellular Agriculture", Front. Sustain. Food Syst. doi: 10.3389/fsufs.2019.00043). *Litopenaeus vannamei* (formerly *Penaeus vannamei*; a.k.a. whiteleg shrimp/Pacific white shrimp/king prawn) and *Penaeus monodon* (a.k.a. giant tiger prawn! Asian tiger shrimp) are predicted to have 1.66 (*vannamei*)/2.6 (*monodon*) Gb genome size and 44 pseudochromosomes (*vannamei*)/88 chromosomes (Zhang et al. (2019) Nature Communications 10:356 and Yuan et al. (2017) J. Marine Genomics doi.org/10.1016/j.margen.2017.12.006). These large genome sizes and the presence of a significant number of genomic repeats makes sequencing and assembly of prawn/shrimp genomes difficult (Yuan et al. (2017) J. Marine Genomics doi.org/10.1016/j.margen.2017.12.006 and Zhang et al. (2019) Nature Communications 10:356). In addition, the few genomic library search engines available for prawn/shrimp are not robust or well-annotated (Shrimp GPAT; Shrimp EST project; NCBI Taxonomy project). Furthermore, genome studies on shrimp/prawn have focused at genome evolution (Yuan et al. (2017) Mar Biotechnol 19(1):76-88; Yuan et al. (2017) Mar Drugs 15(7):213), genetic linkage (Yang et al. (2015) Scientific Reports 5: 15612), and developmental genes (Brown et al. (2018) Genome Biol Evol 10(1): 143-156). This situation is further complicated by the inconsistent and/or interchangeable use of "shrimp" and "prawn" for a given species. Nonetheless, there are at least 2,000 recognized species, the most commercially important being those in the genera *Penaeus, Solenocera*, *Metapenaeus*, *Parapenaeus*, *Parapenaeopsis, Metapenaeopsis, Trachypenaeus*, *Protrachypene*, *Xiphopenaeus*, *Hymenopenaeus, Atypopenaeus*, *Eusicyonia, Sicyonia,* and *Litopenaeus.*

Some studies have examined shrimp/prawn species in terms of physical animal growth by body size and weight. Shrimp growth was improved by transducing foreign Tilapia growth hormone genes into shrimp embryos (Arenal et al. (2008) Biotechnol Lett 30(5):845-51). Suppressive subtractive hybridization identified several growth genes related to body weight in *Penaeus monodon,* (Tangprasittipap et al. (2010) Aquaculture 307(1-2): 150-156). Muscle growth genes have been identified in *Penaeus monodon* but no other species (Ngyuen et al. (2016) Aquaculture 464:545-553). However, genetic modification of genes to immortalize or reprogram muscle cells has either not been attempted or has not been successful.

The situation for crab, crayfish, and/or lobster species is similar. For example, the marbled crayfish (*Procambarus virginalis* (previously *Procambarus fallax f. virginalis*)) has a genome size estimated to be larger than that of humans with 276 chromosomes (*i.e.* 3.5 Gbp; Gutekunst et al. (2018) Nature Ecology & Evolution 2:567-573), while the blue crab (*Callinectes sapidus*) genome is about 2 Gbp in size with an unknown number of chromosomes (see IMET Guardians of the Blue Crab website on the internet; 2.35 pg *see* Animal Genome Size Database on internet; and Dolezel et al. (Cytometry (2003) Part A 51A:127-128) who define genome size (bp) = (0.978 X 10⁹) X DNA content (pg). While no lobster genome has been completely sequenced to date, estimates are that the American lobster (*Homarus americanus*) genome is about 4.40 pg/4.3 Gbp or greater (see Animal Genome Size Database website and Gloucester Marine Genomics Institute website, American Lobster Genome, on the internet).

The lack of information regarding shrimp, prawn, crab, crayfish, and/or lobster is arguably largely due to challenges in cell culture. For example, shrimp/prawn cell lines have only been established from the lymphoid organ and ovaries (Tapay et al. (1995) Proc Soc Exp Biol Med 209(1):73-8; Hsu et al. (1995) Aquaculture 136:43-55; US patent number 6143547; George and Dhar (2010) In Vitro Cell Dev Biol Anim 46(9):801-10; Ma et al. (2015) Reviews in Aquaculture 9: 88-98). In addition, shrimp/prawn primary muscle cultures have lasted only up to 12 days (George and Dhar, 2010) and no known primary cultures or cell lines have been described for fat cells. Even in a primary cell culture of Oka organ, haemolymph and early embryos the isolated shrimp cells stopped diving once *in-vitro* cultured (Guo (2017) J. Cell Dev. Biol. Vol. 1 No. 1:3). Attempts to produce lymphoid cell lines from *Penaeus monodon* by induction with SV-T and 12S EIA oncogenes have also been described (Jayesh et al. (2015) Fish and Shellfish Immunology 47(2):655-663), but in penaeid shrimp, no transformed or immortalized cell line has been established despite reports of attempts using lipofection, electroporation and viral vectors (Kwanta et al. (2018) Fish and Shellfish Immunology 81:204-213). The importance of telomerase activity in Penaeus monodon lymphoid cell culture has been discussed (Jayesh et al. (2015) Cytotechnology 68(4):1301-1314) as has the multifactorial interaction of growth factors in crustacean cell lines which are dependent on each cell type (Jayesh et al. (2014) Cytotechnology 67(3):559-571).

Furthermore, attempts to culture fat cells from shrimp, prawn, crab, crayfish, and/or lobster have not been described.

The availability of immortalized cell lines from shrimp, prawn, crab, crayfish, and/or lobster would allow cellular agriculture companies to progress to the next steps of cellular agriculture (Rubio et al., (2019) "Cell-Based Fish: A Novel Approach to Seafood Production and an Opportunity for Cellular Agriculture", Front. Sustain. Food Syst. doi: 10.3389/fsufs.2019.00043). Rather than the time consuming process of isolating primary cells, food regulatory and research groups would then have a ready source of cell lines. This would facilitate understanding shrimp, prawn, crab, crayfish, and/or lobster viral diseases, testing for the presence of those viruses, and developing treatments (Ma et al. (2015) Reviews in Aquaculture 9: 88-98). Consequently, there remains a need for immortalized muscle and fat cell lines from shrimp, prawn, crab, crayfish, and/or lobster. The disclosure presented herein addresses this need.

### SUMMARY OF THE INVENTION

The present invention is directed to renewable crustacean stem cell lines and to a method for producing shrimp or prawn renewable muscle stem cells lines as defined in the appended claims.

### BRIEF DESCRIPTION OF THE DRAWINGS

**Figure 1** depicts shrimp explants in culture media immediately after sectioning. **A:** explants in T75 flask; **B:** explants in petri dish.
**Figure 2** shows explant cell growth on day 7.
**Figure 3** shows brightfield imaging on Ti-2 Nikon microscope of cells diluted at a 1:3 ratio after reaching 80-90% confluence. **A:** x20 magnification; **B:** x20 magnification.
**Figure 4** shows myofibers beginning to form 3 days after generation of a suspension culture, x40 magnification.
**Figure 5** shows myofibers ready for harvesting after 7 days growth, x40 magnification.
**Figure 6** shows histology of tail segments where muscle stem cells are isolated from; **A:** the final body segment; **B:** increased magnification view of the final body segment; **C:** the middle wing of the tail; **D** increased magnification view of the middle wing; **E:** the side wing of the tail; **F:** increased magnification view of the side wing; **G:** histology of midline juvenile shrimp; muscle regions are outlined in black.

### DETAILED DESCRIPTION OF THE INVENTION

Throughout this disclosure, the term "a" or "an" entity refers to one or more of that entity; for example, "a polynucleotide," is understood to represent one or more polynucleotides. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art to whom this disclosure is directed. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; The Oxford Dictionary Of Biochemistry And Molecular Biology, Revised, 2000, Oxford University Press; Takahashi et al. (2007) Cell 131:861-872; and Yu et al. (2007) Science 21; 318(5858):1917-20 provide one of skill with a general dictionary of many of the terms used in this disclosure.

Units, prefixes, and symbols are denoted in their Systeme International de Unites (SI) accepted form. Numeric ranges are inclusive of the numbers defining the range. The headings provided herein are not limitations of the various aspects of the disclosure, which can be had by reference to the specification as a whole.

Recitation of ranges of values herein are merely intended to serve as a shorthand method of referring individually to each separate value falling within the range, unless otherwise indicated herein, and each separate value is incorporated into the specification as if it were individually recited herein. Where a specific range of values is provided, it is understood that each intervening value is intended to be included therein, and all smaller subranges are also included.

Disclosed herein are comestible shrimp, prawn, crab, crayfish, and/or lobster products as well as new and improved methods for generating muscle and/or fat tissue *in vitro* from a cell source for use in the comestible shrimp, prawn, crab, crayfish, and/or lobster products. Such methods are useful for production of shrimp, prawn, crab, crayfish, and lobster muscle tissue, or "meat," without reliance upon extensive farming or wild fishing. Currently, meat from shrimps, prawns, crab, crayfish, and/or lobster is typically only taken from the tail, leg, or claw muscles. The tail, leg, or claw meat is generally sold whole or in large pieces after removal of the exoskeleton; however, shrimp, prawn, crab, crayfish, and/or lobster meat products also include derivatives such as ground meat for inclusion in "meatballs"/"fishballs" and products containing smoked, pureed, seasoned, and/or dried meat.

Also disclosed are *in vitro* produced shrimp, prawn, crab, crayfish, and/or lobster tissues, such as muscle and/or fat tissue, made by any of the methods provided herein, although these do not form part of the presently claimed invention. The cell source may be an adult stem cell, an induced pluripotent stem (iPS) cell, and/or an immortalized cell line. The shrimp and/or prawn cell source can be isolated from a species from the genus *Penaeus, Solenocera, Metapenaeus, Parapenaeus*, *Parapenaeopsis, Metapenaeopsis, Trachypenaeus*, *Protrachypene, Xiphopenaeus*, *Hymenopenaeus*, *Atypopenaeus*, *Eusicyonia, Sicyonia,* and/or *Litopenaeus.* The crab cell source can be isolated from a species from the genus *Chionoecetes, Callinectes, Charybdis, Cancer, Scylla* and/or *Metacarcinus.* The crayfish cell source can be isolated from the genus *Cambarus*, *Jasus, Thenus, Cambarellus, Cambaroides, Atacopsis, Austropotamobius, Astacus, Procambarus, Orconectes, Faxonella*, *and Pacifastacus*. The lobster cell source can be isolated from a species from the genus *Acanthacaris, Eunephrops*, *Hominarinus*, *Homarus*, *Metanephrops*, *Nephropides, Nephrops*, *Nephropsis*, *Thaumastocheles, Thaumastochelopsis, Thymopides, Thymops,* or *Thymopsis.*

### Isolation of primary muscle and fat cells from crustaceans

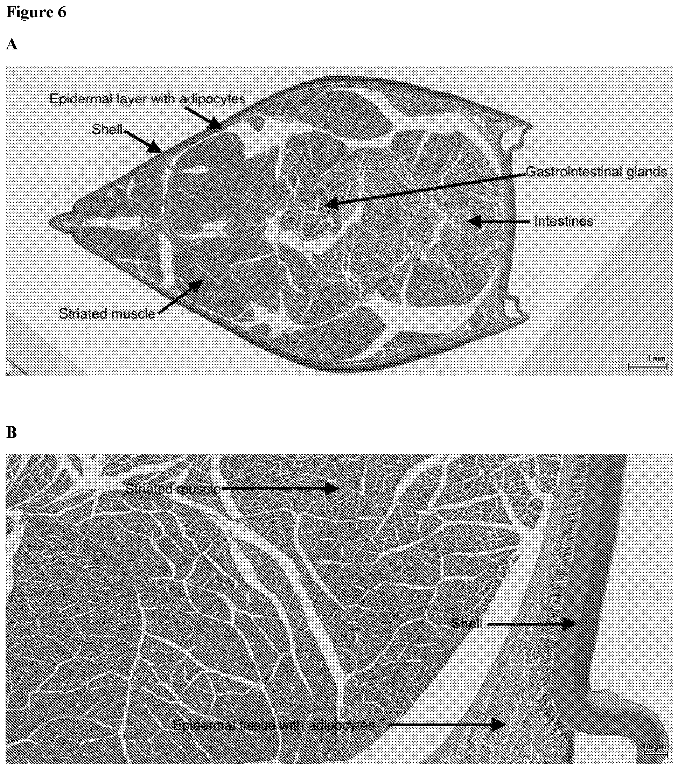

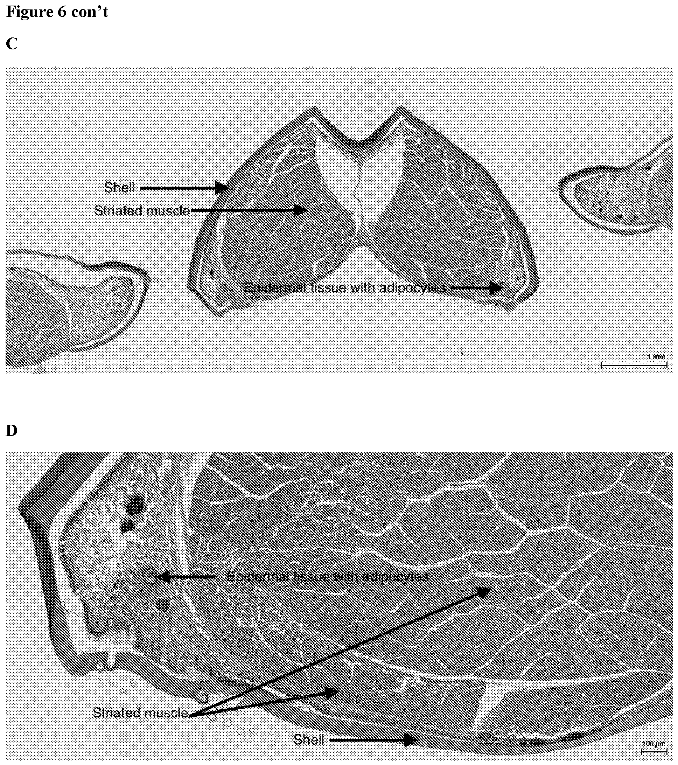

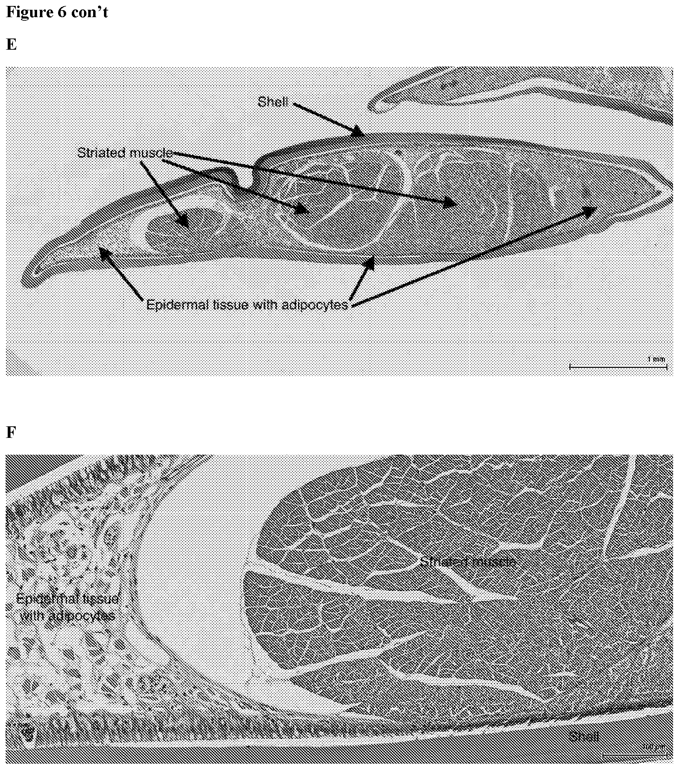

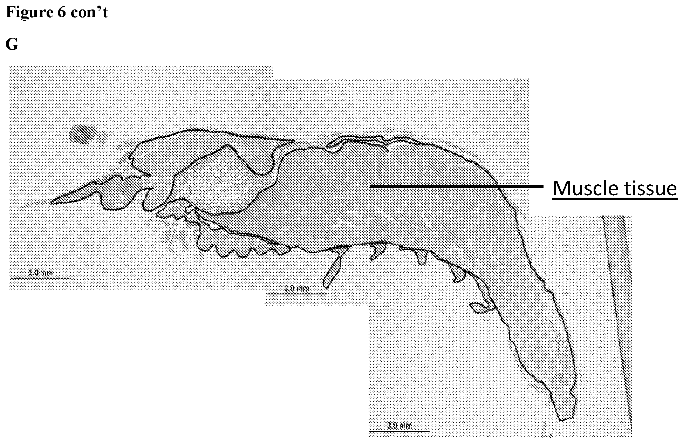

A method of producing shrimp, prawn, crab, crayfish, and/or lobster meat products for consumption by developing renewable muscle and/or fat stem cell lines is disclosed herein. The method can be accomplished by isolating muscle and/or fat cells from various shrimp, prawn, crab, crayfish, and/or lobster species and culturing them *in vitro.* For example, Figure 6 illustrates the histology of shrimp tail segments from which stem cells are isolated.

Explants from freshly sacrificed shrimps, prawns, crabs, crayfish, and/or lobsters can be separated into small pieces. Typically the pieces are at least 0.1 mm in size and can be 0.25 mm, 0.5 mm, 0.75 mm, 1 mm, 1.25 mm, 1.5 mm, 1.75 mm, 2 mm, 2.25 mm, 2.5 mm, 2.75 mm, 3 mm, 3.25 mm, 3.5 mm, 3.75 mm, 4 mm, 4.25 mm 4.55 mm, 4.75 mm, 5 mm or any number between 0.1 mm and 5 mm. Explants are used to seed cell culture media for cell culturing.

When used, explants are incubated in cell culture media, such as Grace's insect media, DMEM high glucose (HYCLONE^{™} SH30022.01, Fischer Scientific, Waltham MA), NUTRISTEM@ MSC XF supplement mix (Biological Industries, Cromwell, CT), NUTRISTEM@ MSC XF basal media (Biological Industries, Cromwell, CT), MYCULT^{™} SF expansion human 10x (STEMCELL TECHNOLOGIES^{™}, Cambridge, MA) HYCLONE^{™} media for undifferentiated mesenchymal stem cells (SH30879.01, GE Lifescience, Boston, MA), HYCLONE^{™} stem cell 10x Supplement (HYCLONE^{™} SH30878.02, Fischer Scientific, Waltham MA), Leibovitz's-15 (L-15) media (ThermoFisher, Waltham, MA), M199 media (ThermoFisher, Waltham, MA), MPS media (ThermoFisher, Waltham, MA), Pj-2 media, NCTC 135 media (ThermoFisher, Waltham, MA), MM insect media (SIGMA-ALDRICH^{®}, St. Louis, MO), or TC 100 media ((ThermoFisher, Waltham, MA), and combinations thereof. The cell culture media may additionally comprise 10% fetal bovine serum (FBS) and/or 5% Penicillin/Streptomycin (PS). Fresh media is added periodically, preferably every 2-3 days.

Optionally, explants may be periodically rinsed with about 5% penicillin-streptomycin filtered Phosphate-Buffered Saline (PBS; 0.137 M NaCl, 0.0027 M KCl, 0.01 M Na₂HPO₄, 0.0018 M KH₂HPO₄, pH 7.4) without further removal of any cells shed from the explants during the rinsing process. Preferably, the rinsing occurs every 2-3 days until explants are removed from the culture media.

The explant/cell cultures may be incubated at 28°C without carbon dioxide. About 7 days after culturing has begun, explants may be removed by centrifugation, filtration, magnetic beads, and/or other means known to those of skill in the art, leaving behind a large population of single cells. The identity of muscle stem cells in the population can be determined by morphological identification and/or PCR amplification of muscle genes. The identity of fat cells can also be determined by morphological identification and/or PCR amplification of adiposespecific genes. Cultures may be routinely tested for mycoplasma and/or other pathogens using standard techniques such as direct growth on broth/agar, specific DNA staining, PCR amplification, ELISA, RNA labeling and enzymatic procedures (e.g. enzymatic conversion of ADP to ATP, *etc*.), PLASMOTEST^{™} (InvivoGen, San Diego, CA), BAM 4/LST-MUG (Feng *et al.* (2002) available from the fda.gov/food/laboratory-methods-food website, chapters 4 and 5), and combinations thereof.

After removal of explants, the remaining cell population may be divided, fresh cell culture media added and incubation continued. Fresh media may be added periodically, for example about every 2-3 days. When the cells are nearing confluence, preferably about 70%, 80%, 90%, 95%, 99%, or any value in between, or the cell population is about 8 million cells in 10 ml media, the cells are again divided and diluted at a 1:3 ratio. The culturing process can include slowly reducing the amount of penicillin-streptomycin from 5% to 2% and then to 0% prior to cryopreservation of the cells.

Cells can be cryopreserved after passage 2 using standard cryopreservation conditions and standard cryopreservation media (see, for example, protocols and reagents available on the internet from Thermo Fisher Scientific, Nippon Genetics, and ATCC). For example, cells can be briefly centrifuged (e.g. about 5 minutes) at 1000 x g, supernatant removed, and cells resuspended in 1 ml of Bambanker freezing media in cryotubes prior to storage at -80°C.

### Differentiation into Myofibers

Approximately 2-3 million primary muscle cells, immortalized muscle stem cells, and/or reprogramed cells can be added to appropriate culture media. Appropriate culture media includes DMEM high glucose (HYCLONE^{™}, SH30022.01; Fisher Scientific, Waltham, MA), Grace's insect media, DMEM high glucose (HYCLONE^{™} SH30022.01, Fischer Scientific, Waltham MA), NUTRISTEM@ MSC XF supplement mix (Biological Industries, Cromwell, CT), NUTRISTEM^{®} MSC XF basal media (Biological Industries, Cromwell, CT), MYOCULT^{™} SF expansion human 10x (STEMCELL TECHNOLOGIES^{™}, Cambridge, MA) HYCLONE^{™} media for undifferentiated mesenchymal stem cells (SH30879.01, GE Lifescience, Boston, MA), HYCLONE^{™} stem cell 10x Supplement (HYCLONE^{™} SH30878.02, Fischer Scientific, Waltham MA), and combinations thereof. The culture media can include augmentation with inactivated fetal bovine serum and/or penicillin-streptomycin. Cell cultures are preferably incubated at 28°C with stirring. Typically, myofibers begin to form 3 days after initiation of the suspension culture. Media can be changed periodically (e.g. every 3 days) by centrifuging the cells/developing myofibers, replacing the media supernatant, and resuspending the cells/developing myofibers. Myofibers are generally harvested 7 days after the suspension culture is initiated.

### Immortalization of isolated primary muscle and/or fat cells

Growth genes specific to muscle and/or fat cells identified from sequencing can be used to immortalize isolated muscle and/or fat cells. RNA sequencing is performed and analyzed using standard techniques (see, for example, Kukurba and Montgonery (2015) Cold Spring Harb Protoc 2015(11):951-969). These genes include, for example, MyoD and SMARCD3, Pax3, Pax7 (reviewed in Chal and Pourquié (2017) Development 144:2104-2122), myosin-1, integrin alpha-7, cadherin-15, myogenin, growth hormone and insulin-like growth factor, myostatin and growth differentiation factor, crustacean hyperglycemic hormone, and myosin heavy chain (Jung et al. (2013) Reviews in Aquaculture 5, 77-110; Ngyuen et al. (2016) Aquaculture 464: 545-553; Okita et al. (2011) Nat Methods 8: 409-412). Non-integrating immortalization methods such as recombinant adenoviral vectors (adm); non-integrative lentiviral vectors like lentiflash particles (vectalys), inducible lentiviral vectors (Bar-Nur et al. (2018) Stem Cell Reports 10:1505-1521), and integrase-deficient lentiviral vectors (Chick et al. (2012) Human Gene Therapy 23:1247-1257); and/or mini circles (Kim et al. (2017) Stem Cell Research 23:87-94) are used to integrate and overexpress the growth genes of interest. Adenoviral vectors include an Ad5-derived, E1A-deleted adenoviral vector expressing the fulllength murine *MyoD* cDNA under the transcriptional control of Rous sarcoma virus LTR (Lattanzi et al. (1998) J Clin. Invest. 101(10):2119-2128); Ad-MyoD, Ad-m-MYOD1 (Vector Biolabs, Cat. No 1492, ADV-265351); human type-5 adenovirus (Suehiro et al. (2010) FEBS Letters 584:3545-3549) and white sturgeon adenovirus (WSAdV-1 (Hendrick et al. (1985) Can J Fish Aquat Sci 42:1321-1325; Hendrick et al. (1990) Dis Aquat Org 8:39-44). An example of non-integrative lentiviral vectors is commercially available lentiflash particles (Vectalys); this includes the customizable pRLP-MS2 plasmid (Vectalys, Toulouse, France), where a muscle growth gene, as mentioned above, is cloned into the vector and is transduced along with pRLP-MCP and VSVG plasmids. Alternatively, inducible lentiviral vectors like tetOP-MyoD and M2rtTA are co-expressed in the isolated muscle cells (Bar-Nur *et al.* (2018)). Similarly, suitable integrase-deficient lentiviral plasmids can be constructed according to Chick *et al.* (2012), including a combination of pHR'SIN-cPPT-SFFV-eGFP-WPRE, pHR'SIN-cPPT-SFFV-NogoB-WPRE, and pCMV delta R8.74 D64V plasmids. Finally, DNA minicircles expressing hPax7 are cloned according to Kim *et al.* (2017).

Otherwise, chemical immortalization of cells is stimulated by use of telomerase activators such as Cycloastragenol (Sigma, SMB00372-20MG; Fauce et al. (2008) J Immunol. 181(10): 7400-7406), Genistein from Glycine Max (soybean) (Sigma, G6776-5MG; Chau et al. (2007) Carcinogenesis 28(11): 2282-2290), or Resveratrol (Sigma, R5010-100MG; Xia et al. (2008) British Journal of Pharmacology 155: 387-394; Zhai et al. (2016) Oncology Letters 11: 3015-3018).

Isolated primary cells at about 70% confluence are used for transduction. Cells are incubated with adenoviral vector constructs containing the gene of interest are incubated at or around 28°C for an hour and evaluated for transduction by PCR, quantitative PCR and/or FACS after 48-72 hours. LENTIFLASH^{®} particles are incubated at or around 28°C with primary cells for about 12 hours before removal. Optionally, the transduction process is repeated approximately 30 hours after the first transduction (Prel et al. (2015) Mol Ther Methods Clin Dev 21(2): 15039). Inducible lentiviral vectors are incubated with primary cells at or around 28°C for 24 hours before removal and then supplemented at 48 and 72 hours with 4-8 µg/ml polybrene transfection reagent (Sigma-Aldrich) (Bar-Nur *et al.* (2018)). Integrase-deficient lentiviral plasmids are incubated with primary cells for 18 hours at or around 28°C in the presence of 8 µg/ml polybrene (Chick et al. (2012) Human Gene Therapy 23(12):1247-1257). Alternatively, mini circles are transduced into primary cells with GENEIN^{™} transfection kit (MTI-Global Stem, Gaithersburg, Maryland) for a total of 3 times every 3 days as reported by Kim et al. (Stem Cell Research (2017) 23:87-94).

When chemical immortalization is relied upon, the cells are incubated with Cycloastragenol (0.01-1µM, Sigma; Fauce et al. (2008) J Immunol. 181(10): 7400-7406); Genistein (0.5-1µM, Sigma; Chau et al. (2007) Carcinogenesis 28(11): 2282-2290) or Resveratrol (10-50µM, Sigma; Xia et al. (2008) British Journal of Pharmacology 155: 387-394; Zhai et al. (2016) Oncology Letters 11: 3015-3018) for a total of 72 hours or 144 hours (supplemented at 72 hours).

The immortality of primary cells is verified by PCR expression of muscle stem cell and/or growth genes or by the ability of cells to be passaged beyond 10 times. Telomerase activity is assessed by telomerase repeated amplification ELISA.

### Generating iPS cells from isolated primary muscle and fat cells

Primary muscle and/or fat cells, and/or immortalized muscle and/or fat cells can be reprogrammed by transfection with episomal plasmids (see, for example, Chandrobose et al. (2018) Stem Cell Research & Therapy 9:68; Slamecka et al. (2016) Cell Cycle 15(2):234-249) or by integration- and xeno-free mRNA transfection (Lee et al. (2016) Stem Cells International 2016: 6853081).

Episomal plasmids from the Yamanaka cocktail can be used: pCXLEhOct3/4-shp53-F, pCXLE-hSK, pCXLE-hUL, and pCXLE-EGFP (Addgene, Watertown, MA) as well as the Yamanaka cocktail of Oct4, Sox2, Klf4 and Myc (Takahashi et al. (2007) Cell 131:861-872; Okita et al. (2011) Nat Methods 8: 409-412; Rosello et al. (2013) Elife 2: e00036).

Immediately following transfection, cells can be placed in culture media. Appropriate culture media include Grace's Insect Media supplemented with 10% FBS and 2% PS (Ma *et al.,* 2017; George and Dhar, 2010) and/or mesenchymal stem cell media. Media is changed daily and on day 7, cells are seeded onto MATRIGEl^{®} (Corning, Tewksbury, MA) for feeder- free induced pluripotent stem (iPS) cell derivation. Subsequently, the media can be changed to mTeSR1 (STEMCELL TECHNOLOGIES^{™}, Cambridge, MA), optionally supplemented with sodium butyrate. Media can also be supplemented at a later date with small molecules SMC4 cocktail (containing small molecules: PD0325901, CHIR99021, Thiazovivin, and SB431542 (FOCUS Biomolecules, Plymouth meeting, PA)) until initial colonies are formed.

Alternatively, microRNA containing reprogramming microRNAs and mRNAs (mir302a-d, mir367, Oct4, Sox2, Klf4, c-Myc, Lin28; Stemgent Inc., Cambridge, MA) can be transfected as previously described (Lee *et al.,* 2016). Isolated primary muscle cells can be seeded in vitronectin XF coated wells a day prior to transfection. mRNA transfection is repeated daily for a period of time, for example for 11 days, while microRNA transfection is repeated on two occasions, such as day 1 and 5, after seeding.

To confirm that the primary muscle and/or fat cells have been reprogrammed to induced pluripotent cells, known pluripotent genes can be analyzed via quantitative PCR and FACS and the results verified by sequencing. Exemplary pluripotent genes include, but are not limited to, PL10 family genes (Mochizuki et al. (2001) Dev Genes Evol 211(6):299-308); Lv-Vasa (Aflalo et al. (2007) Mol Repro Dev 74:172-177), DEAD family genes (Shukalyuk et al. (2007) Cell Biol Int 31(2):97-108); Pou5f1, klf, sall4, hsp60 (Robles et al. (2011) Zebrafish 8(2): 57-63); VVL, SoxN, dmyc, Luna family genes (Rosello et al. (2013) Elife 2: e00036); and piwi (Alie et al. (2011) Dev Biol 350(1):183-97). Functional tests can then be carried out to verify cell potency. Examples of suitable tests include, but are not limited to, *in vitro* differentiation assays, teratoma formation, karyotype analysis and bisulfite sequencing.

### Generation of Muscle and Fat cells from pluripotent embryonic stem cells

The muscle and/or fat cells may be derived from non-human pluripotent embryonic stem cells, such as cells from the blastocyst stage and fertilized eggs.

Pluripotent embryonic stem cells can be initially cultured essentially as described above. Muscle cells can be differentiated from embryonic stem cells or induced pluripotent stem cells as described in Salani et al. (J Cell Mol Med (2012) 16(7):1353-1364) and Chai and Pourquié (Development (2017) 144:2104-2122). Adipocytes can be differentiated from embryonic stem cells and induced pluripotent stem cells as described in Barberi et al. (PLoS Med (2005) 2(6):e161), Mohsen-Kanson et al. (Stem Cells (2014) 32:1459-1467) and Hafner et al. (Scientific Reports (2016) 6: Article Number 32490).

### EXAMPLES

### Example 1- Isolation of primary muscle stem cells from shrimp/prawns

Live Whiteleg shrimps and Tiger prawns (including adult or postlarval stages) are held on ice for 10 minutes prior to sacrificing. Whole shrimps/prawns are rinsed in 11% sodium hypochlorite for 10 seconds and the last body segment and tail are dissected. The remaining body parts are kept on ice.

The dissected tail and attached segment are transferred to 2% potassium permanganate and sterilized for 10 minutes. The tail and attached segment are then rinsed once in PBS buffer (0.137 M NaCl, 0.0027 M KCl, 0.01 M Na₂HPO₄, 0.0018 M KH₂HPO₄, pH 7.4), once in 2% penicillin-streptomycin filtered PBS, and held in 70% ethanol for 5-10 minutes prior to washing in 5% or 10% penicillin-streptomycin filtered PBS for 5-10 minutes.

Cleaned tails and attached segment is kept on ice along with a separate 10 cm dish containing cell culture media. Five different media are used: (1) Grace's insect media with 10% heat-inactivated fetal bovine serum (HYCLONE^{™} SH30071.03, Fischer Scientific, Waltham MA) and 5% penicillin-streptomycin, (2) DMEM high glucose (HYCLONE^{™} SH30022.01, Fischer Scientific, Waltham MA) with 10% heat-inactivated bovine serum and 5% penicillin-streptomycin, (3) NUTRISTEM@ MSC XF supplement mix (Biological Industries, Cromwell, CT):NUTRISTEM^{®} MSC XF basal media (Biological Industries, Cromwell, CT) with 5% penicillin-streptomycin, (4) DMEM high glucose (HYCLONE^{™} SH30022.01, Fischer Scientific, Waltham MA):MYOCULT^{™} SF expansion human 10x (STEMCELL TECHNOLOGIES^{™}, Cambridge, MA):5% penicillin-streptomycin, and (5) HYCLONE^{™} media for undifferentiated mesenchymal stem cells (SH30879.01, GE Lifescience, Boston, MA): HYCLONE^{™} stem cell 10x Supplement (HYCLONE^{™} SH30878.02, Fischer Scientific, Waltham MA): 5% penicillin-streptomycin.

For adult muscle isolations, the tail is removed and reserved while the opaque muscle is dissected from the body segment. The layer of fat and epidermal tissue is removed.

For postlarval muscle isolation, the head is separated from the body. Legs are removed as much as possible and the body segments are minced initially with an electrical mincer or handheld blender.

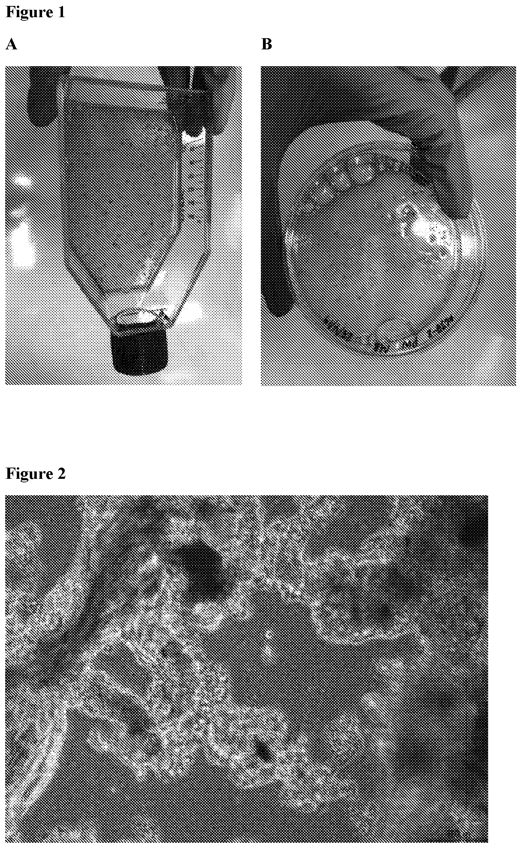

The adult or partially-minced postlarval muscles are manually minced into approximately 1 mm pieces prior to transfer to the prepared 10 cm media containing dish where a second round of mincing is performed. For the adults, muscle is then removed from the tail, minced, transferred to the prepared media dish and minced again. This process is then repeated for the adult winged ends. Upon completion of dissection, the explants and media are transferred to cell culture flasks and/or dishes and incubated at 28°C without CO₂ *(see* Figure 1).

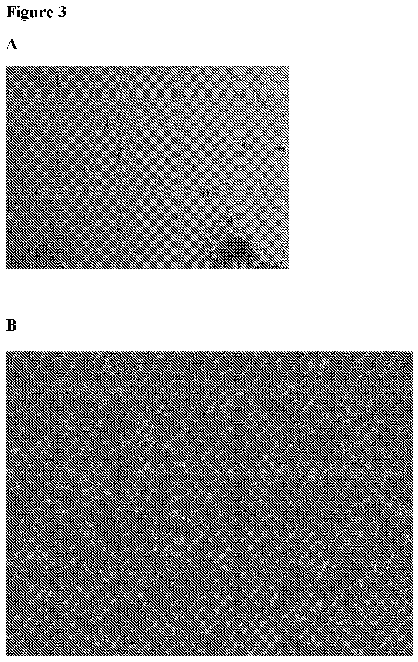

Cultures are maintained by adding fresh media every 2-3 days. Between 3 to 7 days after isolation, explant tissues are removed by gravity separation or centrifugation for 10-30 seconds. The remaining cell population is split according to cell density, usually into 2 T75 flasks, and media added to a final volume of 10-12 ml in each flask *(see* Figure 2). Incubation at 28°C is continued and fresh media added every 2-3 days. When cells number about 8 million and/or are about 80-90% confluent in about 10 ml media, the cell population is again diluted at a 1:3 ratio and incubated under the same conditions continued until passage 2 (see Figure 3). At that time, some cells are centrifuged for 5 minutes at 1000 x g, supernatant removed and cells resuspended in cryotubes in 1 ml of Bambanker freezing media prior to storage at -80°C. Other cells are further cultured in media until at least passage 4. In those cases, the antibiotics present in the culture media is reduced to 2% after passage 2 and further reduced to 0% after passage 4.

To confirm the identity of muscle stem cells, expression of muscle genes is analyzed via quantitative PCR. Here, muscle cell genes such as myostatin and growth differentiation factor, Muscle LIM protein, Alpha skeletal muscle, Myosin heavy chain, myosin-1, Pax3, Pax7, integrin alpha-7, cadherin-15, and myogenin (Jung et al. (2013) Reviews in Aquaculture 5, 77-110; Ngyuen et al. (2016) Aquaculture 464: 545-553; Okita et al. (2011) Nat Methods 8: 409-412) are targeted. Fat cells are identified by the presence of fatty acid-binding protein (Ngyuen et al. (2016) Aquaculture 464), serum amyloid A (SAA), transmembrane 4 L six family member 1 (TM4SF1) (Jernas et al. (2006) FASEB 20(9):1540-1542), or a combination of surface antigens CD44, CD45, and CD105 (Wang et al. (2017) Exp Ther Med 13(3):1039-1043).

Sterility of cell lines is determined by the absence of Mycoplasma. This is tested using commercially available kits such as Cell Culture Contamination Kit, MYCOFLUOR^{™} Mycoplasma Detection Kit, MYCOSEQ^{™} Mycoplasma Detection Kit (ThermoFisher Scientific), MycoAlert PLUS detection kit, PYROGENE^{™} Recombinant Factor C Endpoint Fluorescent Assay (Lonza) and/or FTA Sample Collection Kit for PCR-based Mycoplasma Detection Service (ATCC).

### Example 2 - Differentiation into myofibers

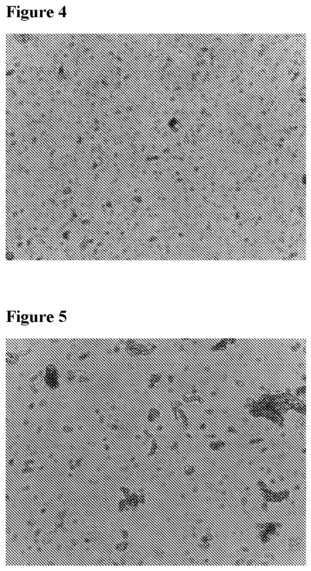

Myofiber differentiation is accomplished by adding approximately 2-3 million primary or immortalized adult muscle stem cells to DMEM high glucose (HYCLONE^{™} SH30022.01, Fischer Scientific, Waltham MA) media with 10% heat-inactivated bovine serum and 5% penicillin-streptomycin. Cells are incubated at 28°C with stirring on a magnetic stirrer set at speed 3 (IKA RCT basic). Media is changed every 3 days by centrifuging fibers at 1000 x g for 5 minutes, removing the used media and replacing it with fresh media. Myofibers begin to form about 3 days after initiation of a suspension culture *(see* Figure 4) and are ready for harvest after 7 days (*see* Figure 5).

### Example 3 - Immortalization of isolated primary muscle and fat cells

Non-integrating immortalization methods can be used to integrate and overexpress growth genes of interest. These genes include for example, MyoD and SMARCD3, Pax3, Pax7 (reviewed in Chal and Pourquié (2017) Development 144:2104-2122), myosin-1, myogenin, integrin alpha-7, cadherin-15, growth hormone and insulin-like growth factor, myostatin and growth differentiation factor, crustacean hyperglycemic hormone, and myosin heavy chain (Jung *et al.* (2013) doi.org/10.1111/raq.12005; Ngyuen et al. (2016) Aquaculture 464:545-553; Okita et al. (2011) Nat Methods 8: 409-412). However, chemical immortalization can also be accomplished using activators such as Cycloastragenol (Fauce et al. (2008) J Immunol. 181(10): 7400-7406), Genistein from Glycine Max (soybean) (Chau et al. (2007) Carcinogenesis 28(11): 2282-2290), or Resveratrol (Xia et al. (2008) British Journal of Pharmacology 155: 387-394; Zhai et al. (2016) Oncology Letters 11: 3015-3018) to increase telomerase activity.

Immortality of primary cells is verified by PCR expression of muscle stem cell and/or growth genes or the ability of cells to be passaged beyond 10 times. When chemical immortalization is used, the telomerase repeated amplification ELISA assay is used to assess the increase in activity and length of telomerase.

### Example 4 - Generating iPS cells from isolated primary muscle and fat cells

Primary muscle and fat cells are reprogrammed by episomal plasmids essentially as described by Chandrabose *et al.,* 2018 or by integration- and xeno-free mRNA transfection as described by Lee *et al.,* 2016.

Episomal plasmids (Addgene) targeting factors from the sequencing results for shrimp are used. In addition, the Yamanaka cocktail of Oct4, Sox2, Klf4 and Myc is also used. Grace's Insect Media supplemented with 10% FBS and 2% PS (Ma *et al.,* 2017; George and Dhar, 2010) or mesenchymal stem cell media is used immediately following transfection. Media is changed daily. On day 7, cells are seeded onto Matrigel (Corning) for feeder- free iPS derivation. On the next day, the media is changed to mTeSR1 (Stem Cell Technologies), supplemented with sodium butyrate. On day 12, sodium butyrate is no longer added, and instead replaced with small molecules SMC4 cocktail (containing small molecules: PD0325901, CHIR99021, Thiazovivin, and SB431542 (FOCUS Biomolecules)) until initial colonies are formed.

Alternatively, microRNA containing reprogramming microRNAs and mRNAs (mir302a-d, mir367, Oct4, Sox2, Klf4, c-Myc, Lin28; Stemgent Inc., MA) are transfected as described previously (Lee *et al.,* 2015). Isolated primary muscle cells are seeded in a vitronectin XF coated well a day prior to transfection. mRNA transfection is repeated for 11 days from day 2 while microRNA transfection is repeated on day 1 and 5 after seeding.

To confirm that the primary muscle and fat cells are reprogrammed to induced pluripotent cells, known pluripotent genes, verified by sequencing, are analyzed via quantitative PCR and FACS. Exemplary pluripotent genes include, but are not limited to, PL10 family genes (Mochizuki et al. (2001) Dev Genes Evol 211(6):299-308); Lv-Vasa (Aflalo et al. (2007) Mol Repro Dev 74:172-177), DEAD family genes (Shukalyuk et al. (2007) Cell Biol Int 31(2):97-108); Pou5f1, klf, sall4, hsp60 (Robles et al. (2011) Zebrafish 8(2): 57-63); VVL, SoxN, dmyc, Luna family genes (Rosello et al. (2013) Elife 2: e00036); and piwi (Alie et al. (2011) Dev Biol 350(1):183-97). Functional tests such as *in vitro* differentiation assays, teratoma formation, karyotype analysis and bisulfite sequencing are carried out to verify the potency of the cells.

Upon confirmation of the presence of iPS cells, these cells can be frozen upon 80% confluence.

### Example 5 - Isolation and expansion of adipose stem cells from shrimp, crab, crayfish, and lobster

Following sacrifice and cleaning of shrimps/prawns, crab, crayfish, or lobster as described above in paragraphs [045], [046] and/or [049], the head of the shrimp/prawn, crab, crayfish, or lobster is separated from the body and tail segments. The legs are also separated from the body of the shrimp/prawns, crab, crayfish, or lobster. The shell is removed from the segments; and the adipose and epidermal tissue is peeled off the tail and body segments with a disposable scalpel and forceps. The adipose and epidermal tissues are washed extensively in PBS containing 5% Penicillin/Streptomycin. The tissue is then digested in Collagenase Type I and minced with scalpels as described in Bunnell et al. (Methods 2008; 45(2):115-120). The adipose stem cells are separated from epidermal or stromal cells as described by Bunnell et al. (Methods 2008; 45(2):115-120).

The isolated cells are resuspended in alpha-MEM (Mediatech, Herndon, VA) supplemented with 20% FBS, 1% L-glutamine (Mediatech) and 1% penicillin/streptomycin. The cell suspension is filtered through a 70 µm cell strainer as described in Bunnell et al. (Methods 2008; 45(2):115-120). The cells are cultured in a lysine coated culture plate (Bunnell et al. Methods 2008; 45(2):115-120) and incubated at 28°C (George and Dhar (2010) In Vitro Cell Dev. Biol. - Animal 46:801-810).

Culture media is changed and cells are washed 72 hours after plating as described in Bunnell et al. (Methods 2008; 45(2):115-120). To maintain cells afterwards, culture media is changed every second day until cells reach 80-90% confluence. The cells can be harvested or differentiated once they reach 80-90% confluence, as described in Bunnell et al. (Methods 2008; 45(2):115-120).

### Example 6 - Isolation of primary muscle stem cells from crab, crayfish, and lobster

Following sacrifice and cleaning of crabs or lobster on ice as described in [045] and [046], muscle of crab claws, legs and main body are isolated and processed as described in [045] - [047]. The crab muscle stem cells are cultured at 20-24°C as described in Sashikumar and Desai (Cytotechnology (2008) 56:161-169).

For crayfish, and lobsters, muscle from the claws, legs and tail is isolated, processed and cultured as described in [045] - [047]. Crayfish muscle stem cells are cultured at 27°C as described in Neumann et al. (In Vivo (2000) 14(5): 691-8). Lobster muscle stem cells are cultured at saturated humidity at 5°C as described in Stepanyan et al. (Chemical Senses (2004) 29(3):179-187) or the temperature at which the lobsters were kept while alive.

## Claims

1. A renewable crustacean stem cell line comprising muscle and/or fat stem cells, wherein the muscle stem cells and/or the fat stem cells are selected from the group consisting of shrimp, prawn, lobster, crab, and crayfish.

2. The renewable crustacean stem cell line of claim 1, wherein the muscle stem cells and/or fat stem cells are shrimp or prawn muscle stem cells and/or fat stem cells.

3. The renewable crustacean stem cell line of claim 1, wherein the muscle stem cells and/or fat stem cells are crab muscle stem cells and/or fat stem cells.

4. The renewable crustacean stem cell line of claim 1, wherein the muscle stem cells and/or fat stem cells are lobster muscle stem cells and/or fat stem cells.

5. A method for producing a shrimp or prawn renewable muscle stem cell line comprising:
(a) incubating live adult or postlarval shrimp or prawns on ice for 10 minutes prior to sacrificing;
(b) rinsing the whole shrimp or prawns in 11% sodium hypochlorite for 10 seconds prior to dissecting the last body segment and tail;
(c) sterilizing the dissected tails and attached segments in 2% potassium permanganate solution for 10 minutes, then rinsing once in phosphate buffered saline (PBS; 0.137 M NaCl, 0.0027 M KCl, 0.01 M Na₂HPO₄, 0.0018 M KH₂HPO₄, pH 7.4), then once in 2% penicillin-streptomycin filtered PBS and then holding in 70% ethanol for 5-10 minutes prior to washing in 5% or 10% penicillin-streptomycin filtered PBS for 5-10 minutes;
(di) for adult muscle isolations, removing and reserving the tail and dissecting opaque muscle from the body segment and removing fat and epidermal tissue; or
(dii) for postlarval muscle isolation, separating the head from the body, removing legs and mincing the body segments with an electrical mincer or handheld blender;
(e) then manually mincing the adult or postlarval muscle into pieces approximately 1.0 mm in size prior to transfer into a 10 cm dish containing one of the following cell culture media:
i. Grace's insect media with 10% heat-inactivated fetal bovine serum and 5% penicillin-streptomycin;
ii. DMEM high glucose with 10% heat-inactivated bovine serum and 5% penicillin-streptomycin;
iii. NUTRISTEM^{®} MSC XF supplement mix : NUTRISTEM^{®} MSC XF basal media with 5% penicillin-streptomycin;
iv. DMEM high glucose : HYCLONE^{™} : MYOCULT^{™} SF expansion human 10x : 5 % penicillin-streptomycin; or
v. HYCLONE^{™} media for undifferentiated mesenchymal stem cells : HYCLONE^{™} stem cell 10x Supplement : 5 % penicillin-streptomycin,
and mincing again;
(f) for adult tissue, removing muscle from the reserved tail, mincing, transferring into the 10cm dish containing culture media and mincing again;
(g) transferring the minced tissue and cell culture media to cell culture flasks and/or dishes and incubating at 28°C without CO₂ for 3-7 days with fresh media added every 2-3 days;
(h) removing tissues by gravity separation or centrifugation for 10-30 seconds then splitting the remaining cell population by density, adding fresh media to a final volume of 10-12 ml per T75 flask, and continuing incubation at 28°C with fresh cell culture media added every 2-3 days; and
(i) when cells number about 8 million in 10 ml of culture media again dividing said cells and diluting said cells at a 1:3 ratio and incubating under the same conditions until passage 2;
(j) centrifuging a portion of the cells for 5 minutes at 1000 x g, removing supernatant and resuspending the cells in cryotubes in 1ml of Bambanker freezing media prior to storage at -80°C; and
(k) culturing the remaining portion of cells in cell culture media until at least passage 4 with antibiotics present in the culture media reduced to 2% after passage 2 and further reduced to 0% after passage 4.

6. The method of claim 5, wherein the shrimp are Whiteleg shrimp and/or the prawns are Tiger Prawns.

7. The method of claim 5 or 6, wherein the method further comprises:
(1) confirming the identity of muscle stem cells by expression of muscle genes, such as myostatin, growth differentiation factor, Muscle LIM protein, alpha skeletal muscle, myosin heavy chain, mysin-1, Pax3, Pax7, integrin alpha-7, cadherin-15 and myogenin.

## Patentansprüche

1. Regenerative Krustentier-Stammzell-Linie umfassend Muskel- und/oder Fettstammzellen, wobei die Muskelstammzellen und/oder die Fettstammzellen ausgewählt werden aus der Gruppe bestehend aus Garnelen, Riesengarnelen, Hummer, Krebsen und Langusten.

2. Regenerative Krustentier-Stammzell-Linie nach Anspruch 1, wobei die Muskelstammzellen und/oder die Fettstammzellen Muskelstammzellen und/oder Fettstammzellen aus Garnelen oder Riesengarnelen sind.

3. Regenerative Krustentier-Stammzell-Linie nach Anspruch 1, wobei die Muskelstammzellen und/oder die Fettstammzellen Muskelstammzellen und/oder Fettstammzellen aus Krebsen sind.

4. Regenerative Krustentier-Stammzell-Linie nach Anspruch 1, wobei die Muskelstammzellen und/oder die Fettstammzellen Muskelstammzellen und/oder Fettstammzellen aus Hummer sind.

5. Verfahren zum Produzieren einer regenerativen Muskelstammzellen-Linie aus Garnelen oder Riesengarnelen umfassend:
(a) Inkubation lebender adulter oder postlarvaler Garnelen oder Riesengarnelen auf Eis für 10 zehn Minuten vor dem Töten;
(b) Spülen der ganzen Garnelen oder Riesengarnelen in 11%igem Natriumhypochlorit für 10 Sekunden vor dem Zerlegen des letzten Körpersegments und des Schwanzes;
(c) Sterilisieren der zerlegten Schwänze und anhängender Segmente in 2%iger Kaliumpermanganatlösung für 10 Minuten, dann einmaliges Spülen in phosphatgepufferter Kochsalzlösung (PBS; 0,137 M NaCl, 0,0027 M KCl, 0,01 M Na₂HPO₄, 0.0018 M KH₂HPO₄, pH 7,4), dann einmal in 2%iger Penicillin-Streptomycin-gefilterter PBS und dann Einlegen in 70%igem Ethanol für 5-10 Minuten vor dem Waschen in 5%iger oder 10%iger Penicillin-Streptomycin-gefilterter PBS für 5-10 Minuten;
(di) für die Isolierung adulter Muskeln Entfernen und Aufbewahren des Schwanzes und Herausschneiden von opaken Muskeln aus dem Körpersegment und Entfernen von Fett- und Hautgewebe;
(dii) für die Isolierung postlarvaler Muskeln Abtrennen des Kopfes von dem Körper, Entfernen der Beine und Zerkleinern der Körpersegmente mit einem elektrischen Fleischwolf oder Handmixer;
(e) dann manuelles Zerkleinern der adulten oder postlarvalen Muskeln in etwa 1,0 mm große Stücke vor dem Überführen in eine Schale ∅ 10 cm, die eines der folgenden Zellkulturmedien enthält:
i. Grace's Insect Medium mit wärme-inaktiviertem fetalem Kälberserum und 5%igem Penicillin-Streptomycin;
ii. DMEM High Glucose mit 10%igem wärme-inaktiviertem Kälberserum und 5%igem Penicillin-Streptomycin;
iii. NUTRISTEM^{®} MSC XF Supplement-Mix : NUTRISTEM^{®} MSC XF Basal-Medium mit 5%igem Penicillin-Streptomycin;
iv. DMEM High Glucose : HYCLONE^{™} : MYOCULT^{™} Expansion Human 10x : 5%iges Penicillin-Streptomycin; oder
v. HYCLONE^{™} Medium für undifferenzierte mesenchymale Stammzellen : HYCLONE^{™} Stammzellen 10x Supplement : 5%iges Penicillin-Streptomycin,
und erneutes Zerkleinern;
(f) für adultes Gewebe Entfernen des Muskels von dem aufbewahrten Schwanz, Zerkleinern, Überführen in die Schale ∅ 10 cm, die Kulturmedien enthält, und erneutes Zerkleinern;
(g) Überführen des zerkleinerten Gewebes und der Zellkulturmedien in Zellkulturflaschen und/oder -schalen und Inkubieren bei 28°C ohne CO₂ für 3-7 Tage unter Zugabe von frischen Medien alle 2-3 Tage;
(h) Entfernen von Gewebe durch Schwerkrafttrennung oder Zentrifugieren für 10-30 Sekunden, dann Aufteilung der verbleibenden Zellpopulation nach Dichte, Zugabe von frischen Medien bis zu einem Endvolumen von 10-12 ml pro T75-Flasche und Fortsetzen der Inkubation bei 28°C unter Zugabe von frischen Zellkulturmedien alle 2-3 Tage; und
(i) wenn die Zellen etwa 8 Millionen in 10 ml Kulturmedium betragen, erneutes Teilen der Zellen und Verdünnen der Zellen in einem Verhältnis von 1:3 und Inkubieren unter gleichen Bedingungen bis Durchgang 2;
(j) Zentrifugieren eines Teils der Zellen für 5 Minuten bei 1000 x g, Entfernen des Überstands und Resuspendieren der Zellen in Kryoröhrchen in ml Bambanker-Gefriermedium vor der Lagerung bei -80°C; und
(k) Kultivieren des verbleibenden Teils der Zellen in einem Zellkulturmedium zumindest bis Durchgang 4, wobei in dem Kulturmedium vorhandene Antibiotika nach Durchgang 2 auf 2% reduziert sind und nach Durchgang 4 weiter auf 0%.

6. Verfahren nach Anspruch 5, wobei die Garnelen Weißfußgarnelen und/oder die Riesengarnelen Tigergarnelen sind.

7. Verfahren nach Anspruch 5 oder 6, wobei das Verfahren ferner umfasst:
(l) Bestätigen der Identität der Muskelstammzellen durch Expression der Muskelgene, wie z.B. Myostatin, Wachstums-Differenzierungsfaktor, Muskel-LIM-Protein, Alpha-Skelettmuskel, schwere Myosinkette, Myosin 1, Pax3, Pax7, Integrin Alpha 7, Cadherin 15 und Myogenin.

## Revendications

1. Une lignée de cellules souches de crustacés renouvelable comprenant des cellules souches musculaires et/ou graisseuses, dans laquelle les cellules souches musculaires et/ou les cellules souches graisseuses sont sélectionnées dans le groupe composé de crevettes, de gambas, de homards, de crabes et d'écrevisses.

2. La lignée de cellules souches de crustacés renouvelable selon la revendication 1, dans laquelle les cellules souches musculaires et/ou les cellules souches graisseuses sont des cellules souches musculaires de crevettes ou de gambas et/ou des cellules souches graisseuses.

3. La lignée de cellules souches de crustacés renouvelable selon la revendication 1, dans laquelle les cellules souches musculaires et/ou les cellules souches graisseuses sont des cellules souches musculaires de crabe et/ou des cellules souches graisseuses.

4. La lignée de cellules souches de crustacés renouvelable selon la revendication 1, dans laquelle les cellules souches musculaires et/ou les cellules souches graisseuses sont des cellules souches musculaires de homard et/ou des cellules souches graisseuses.

5. Un procédé de production d'une lignée de cellules souches musculaires renouvelable de crevettes ou de gambas comprenant :
a) l'incubation de crevettes ou de gambas vivantes adultes ou post-larvaires sur de la glace pendant 10 minutes avant de les sacrifier ;
b) rincer les crevettes entières dans de l'hypochlorite de sodium à 11 % pendant 10 secondes avant de disséquer le dernier segment du corps et la queue ;
c) stériliser les queues disséquées et les segments qui y sont attachés dans une solution de permanganate de potassium à 2 % pendant 10 minutes, puis rincer une fois dans une solution saline tamponnée au phosphate (PBS; 0,137 MNaCl, 0,0027 M KCl, 0,01 M Na₂HPO₄, 0,0018 M KH₂HPO₄, pH 7,4), puis une fois dans du PBS filtré à 2% de pénicilline-streptomycine, puis maintenir dans de l'éthanol à 70 % pendant 5 à 10 minutes avant de laver dans du PBS filtré à 5 % ou 10 % de pénicilline-streptomycine pendant 5 à 10 minutes ;
di) pour l'isolement des muscles adultes, l'ablation et la mise en réserve de la queue et la dissection du muscle opaque du segment du corps et l'élimination de la graisse et du tissu épidermique ; ou
dii) pour l'isolement des muscles post-larvaires, séparer la tête du corps, enlever les pattes et hacher les segments du corps à l'aide d'un hachoir électrique ou d'un mélangeur à main ;
e) hacher ensuite manuellement le muscle adulte ou post-larvaire en morceaux d'environ 1,0 mm avant de les transférer dans une boîte de 10 cm contenant l'un des milieux de culture cellulaire suivants :
i.Milieu: insectes de Grace contenant 10 % de sérum bovin par la chaleur et 5 % de pénicilline-streptomycine ;
ii. DMEM à taux de glucose élevé avec 10 % de sérum foetal bovin inactivé par la chaleur, et 5 % de pénicilline-streptomycine ;
iii. NUTRISTEM^{®} mélange de suppléments MSC XF: Milieu basique NUTRISTEMO MSC XF avec 5% de pénicilline-streptomycine ;
iv. DMEM hyperglycémie : HYCLONE^{™} MYOCULT^{™} SF expansion humaine 10x : 5% pénicilline-streptomycine ; ou
v. HYCLONE ^{™} milieu pour cellules souches mésenchymateuses indifférenciées Supplément 10x de cellules souches HYCLONE^{™} : 5% de pénicilline-streptomycine, et hachage à nouveau ;
f) dans le cas de tissus adultes, enlever le muscle de la queue réservée, hacher, transférer dans la boîte de 10 cm contenant le milieu de culture et hacher à nouveau ;
g) transférer les tissus hachés et des milieux de culture cellulaire dans des flacons et/ou des boîtes de culture cellulaire et incuber à 28°C en l'absence de CO₂ pendant 3 à 7 jours avec ajout de nouveaux milieux tous les 2 à 3 jours ;
h) éliminer les tissus par séparation par gravité ou centrifugation pendant 10 à 30 secondes, puis diviser la population cellulaire restante en fonction de la densité, ajouter un milieu frais à un volume final de 10 à 12 ml par flacon T75 et poursuivre l'incubation à 28°C avec un milieu de culture cellulaire frais ajouté tous les 2-3 jours ;
i) lorsque le nombre de cellules est d'environ 8 millions dans 10 ml de milieu de culture, diviser à nouveau lesdites cellules et les diluer dans un rapport de 1/3 puis les incuber dans les mêmes conditions jusqu'au passage 2 ;
j) centrifuger une partie des cellules pendant 5 minutes à 1000 x g, enlever le surnageant et remettre les cellules dans 1ml de milieu de congélation Bambanker en suspension dans des cryotubes avant de les entreposer à -80°C ;
k) effectuer la culture de la partie restante des cellules dans des milieux de culture cellulaire au moins jusqu'au passage 4 avec des antibiotiques présents dans le milieu de culture, réduit à 2 %, après le passage 2 et à 0 % après le passage 4.

6. Le procédé selon la revendication 5, dans lequel les crevettes sont des crevettes à pattes blanches et/ou ses gambas sont des gambas tigrées.

7. Le procédé selon la revendication 5 ou 6, dans lequel le procédé comprend en outre :
l) confirmer l'identité des cellules souches musculaires par l'expression de gènes musculaires, tels que la myostatine, le facteur de différenciation de croissance, la protéine LIM musculaire, le muscle squelette alpha, la chaîne lourde de myosine, la mysine-l, la Pax3, la Pax7, l'intégrine alpha-7, la cadhérine-15 et la myogénine.
